(19) Europäisches Patentamt
European Patent Office
Office européen des brevets

(11) Veröffentlichungsnummer: **0 152 759**
**B1**

(12) **EUROPÄISCHE PATENTSCHRIFT**

(45) Veröffentlichungstag der Patentschrift: **23.08.89**

(21) Anmeldenummer: **85100350.9**

(22) Anmeldetag: **15.01.85**

(51) Int. Cl.⁴: **C 07 D 275/02**, C 07 D 513/04 // (C07D513/04, 275:00, 209:00), (C07D513/04, 275:00, 237:00)

# (54) Aminoisothiazolverbindungen.

(30) Priorität: **21.01.84 DE 3402024**

(43) Veröffentlichungstag der Anmeldung: **28.08.85 Patentblatt 85/35**

(45) Bekanntmachung des Hinweises auf die Patenterteilung: **23.08.89 Patentblatt 89/34**

(84) Benannte Vertragsstaaten: **CH DE FR GB IT LI**

(56) Entgegenhaltungen:
**EP-A-0 066 389**
**FR-A-1 516 777**

**CHEMICAL ABSTRACTS, Band 54, Nr. 12, 25. Juni 1960, Easton, Pennsylvania, USA; A. ADAMS et al. "Isothiazole: A new mononuclear heterocyclic system", Spalten 12113-12115**
**CHEMICAL ABSTRACTS, Band 51, Nr. 5, 10. März 1957, Easton, Pennsylvania, USA; T.S. GARDNER et al. "The synthesis of compounds for the chemotherapy of tuberculosis. VI. Hydrazine derivatives", Spalten 3610-3611**
**JOURNAL OF THE AMERICAN CHEMICAL SOCIETY, Band 80, Nr. 6, 20. März 1958, Easton, Pennsylvania, USA; R.H. WILEY et al. "Carbamylmaleimides from the Malonamide-Diethyl Oxalate Reaction", Seiten 1385-1388**

(73) Patentinhaber: **BASF Aktiengesellschaft, Carl-Bosch- Strasse 38, D-6700 Ludwigshafen (DE)**

(72) Erfinder: **Eilingsfeld, Heinz, Dr., Pierstrasse 9a, D-6710 Frankenthal (DE)**
Erfinder: **Etzbach, Karl- Heinz, Dr., Bensheimer Ring 9a, D-6710 Frankenthal (DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 152 759 B1

LIBER, STOCKHOLM 1989

**Beschreibung**

Die Erfindung betrifft Verbindungen der Formel I

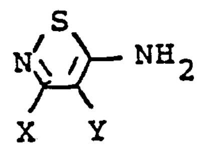

I,

in der

X und Y unabhängig voneinander Carboxyl, $C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxyl)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, durch Chlor, Brom, Nitro oder Cyan substituiertes Phenoxycarbonyl, $C_1$- bis $C_4$-Alkylphenoxycarbonyl, Dimethylphenoxycarbonyl, Trimethylphenoxycarbonyl, Carbamoyl, $C_1$- bis $C_{12}$-Alkylcarbamoyl, Cyclohexylcarbamoxyl, Allylcarbamoyl, 2-($C_1$- bis $C_4$-Alkoxy)-ethylcarbamoyl, 3-($C_1$- bis $C_4$-Alkoxy)-propylcarbamoyl, 2-Phenoxyethylcarbamoyl, 3-Phenoxypropylcarbamoyl, Phenylmethylcarbamoyl, 2-Phenylethylcarbamoyl, Phenylcarbamoyl, durch Chlor, Brom, Nitro oder Cyan substituiertes Phenylcarbamoyl, $C_1$- bis $C_4$-Alkylphenylcarbamoyl, Dimethylphenylcarbamoyl, Trimethylphenylcarbamoyl, $C_1$- bis $C_4$-Dialkylcarbamoyl, Methyl-cyclohexylcarbamoyl, Dicyclohexylcarbamoyl,

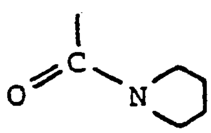 , 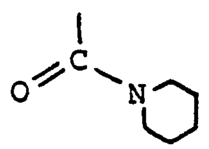 , 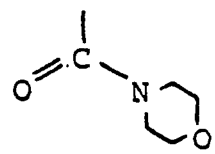 .

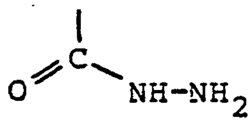 , 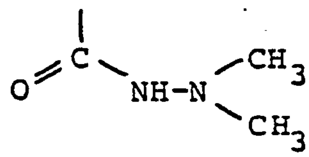 , 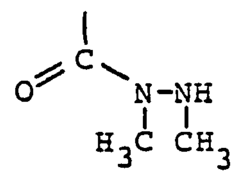 ,

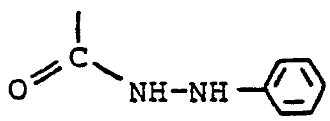 oder 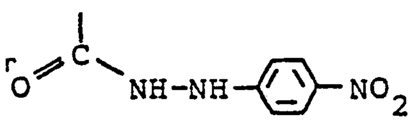 , oder

X und Y zusammen einen Rest der Formel

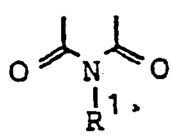 oder 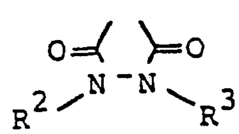

bedeuten, wobei

$R^1$ für Wasserstoff, $C_1$- bis $C_{12}$-Alkyl, Cyclohexyl, Allyl, 2-($C_1$- bis $C_4$-Alkoxyl)-ethyl, 3-($C_1$- bis $C_4$-Alkoxy)-propyl, 2-Phenoxyethyl, 3-Phenoxypropyl, Phenylmethyl, Phenylethyl, Phenyl, $C_1$- bis $C_4$-Alkylphenyl, Dimethylphenyl oder Trimethylphenyl oder jeweils durch Chlor, Brom, Cyan oder Nitro substituiertes Phenyl, $C_1$- bis $C_4$-Alkylphenyl, Dimethylphenyl oder Trimethylphenyl, und

$R^2$ und $R_3$ für Wasserstoff oder $C_1$- bis $C_{12}$-Alkyl stehen, wobei einer dieser Reste auch Phenyl oder durch Chlor, Brom, Cyan oder Nitro substituiertes Phenyl bedeuten kann.

Wenn die Carbamoylverbindungen durch Öffnung des Ringes

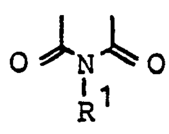

hergestellt werden, bleibt die Gruppe der Formel $-CONHR^1$ erhalten, die zweite Carbamoylgruppe, die entweder X oder Y entspricht, wird mit dem zur Ringspaltung verwendeten Amin gebildet.

Gleiches gilt entsprechend für die Ester, Säure und Hydrazide.

Zur Herstellung der Verbindungen der Formel I kann man Verbindungen der Formel

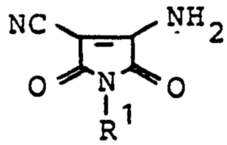

II,

die aus Verbindungen der Formel

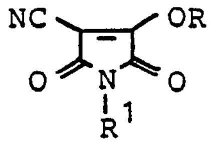

III

durch Umsetzung mit Ammoniak zugänglich sind, mit Dithiophosphorsäureestern umsetzen; man erhält dann zunächst Verbindungen der Formel

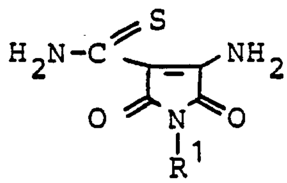

IV,

die nach bekannten Methoden, z. B. durch Umsetzung mit $H_2O_2$ in Eisessig, in die Isothiazolverbindungen überführt werden können.

Verbindungen der Formel III sind analog der in J. Am. Chem. Soc. 80, 1385 (1958) für die Verbindung der Formel

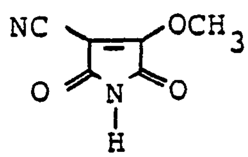

V

und in der DE-OS-3 204 713 für die Verbindung der Formel

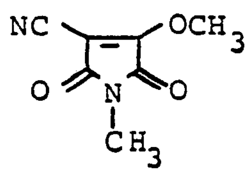

VI

angegebenen Methode erhältlich.

Repräsentative Einzelheiten der Herstellung können den Beispielen entnommen werden, in denen sich Angaben über Teile und Prozente, sofern nicht anders vermerkt, auf das Gewicht beziehen.

Die Verbindungen der Formel I sind diazotierbar und eignen sich daher als Diazokomponenten.

Von besonderer Bedeutung sind Verbindungen der Formel I, bei denen

X = Y = CN oder

X und Y = $CONHB^1$ sind,

wobei die Reste $B^1$ gleich oder verschieden sind und $B^1$ Wasserstoff, $C_1$- bis $C_{12}$-Alkyl oder Phenyl bedeutet.

Weiterhin ist für

X + Y der Rest der Formel 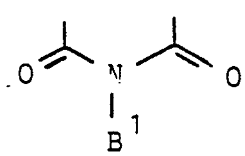 bevorzugt,

wobei $B^1$ die angegebene Bedeutung hat.

Weiter sind Verbindungen der Formel

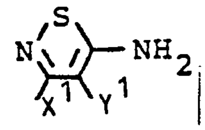

wertvoll, in der einer der Reste $X^1$ und $Y^1$ für den Rest $CONHB^1$ und der andere für Di-($C_1$- bis $C_{12}$-Alkyl)-carbamoyl, Carboxyl, $C_1$- bis $C_{12}$-Alkoxycarbonyl, Phenoxycarbonyl oder einen Rest der Formel

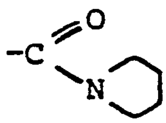 , 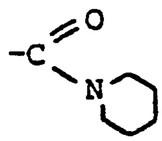 oder 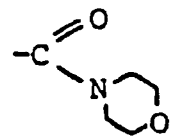

steht und
$B^1$ die angegebene Bedeutung hat.

**Beispiel 1**

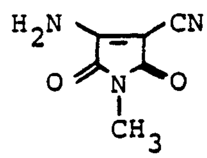

128 Teile 2-cyan-3-methoxy-N-methylmaleinimid werden in 600 Teilen Methylglykol gelöst, anschließend leitet man unter Eiskühlung 13,5 Teile Ammoniak in die Lösung ein. Danach wird noch 3 h bei Raumtemperatur gerührt, der entstandene Niederschlag abgesaugt, mit Methanol und dann mit Wasser gewaschen und getrocknet. Man erhält 90 Teile (83 % der Theorie) 3-Amino-2-cyan-N-methylmaleinimid.
Schmp.: 284°C (Methylglykol), IR (KBr): 3285, 3122 ($NH_2$), 2220 (C≡N), 1788, 1725, 1605 $cm^{-1}$ (C=O)

$C_5H_5N_3O_2$ (151,13)

| | | | | |
|---|---|---|---|---|
| Ber.: | C 47,69 | H 3,33 | N 27,80 | O 21,17 |
| Gef.: | C 47,6 | H 3,5 | N 27,5 | O 21,7 |

**Beispiel 2**

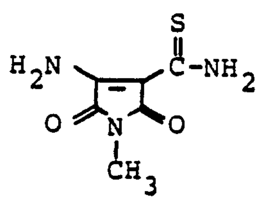

Eine Mischung aus 100 Teilen Phosphorpentasulfid und 450 Teilen Methylglykol wird 0,5 h zum Sieden erhitzt, anschließend werden in diese Lösung 90 Teile 3-Amino-2-cyan-N-methylmaleinimid bei 100°C eingetragen. Die Mischung wird 3 h bei 100°C und dann über Nacht bei Raumtemperatur gerührt. Der entstandene Niederschlag wird abgesaugt, mit Methanol und dann Wasser gewaschen und getrocknet. Man erhält 65,1 Teile (59 % der Theorie) 3-Amino-2-thiocarbamoyl-N-methylmaleinimid.
Schmp.: 282°C (Methylglykol), IR (KBr): 3390, 3280 ($NH_2$), 1760, 1700, 1625 $cm^{-1}$ (C=O)

$C_6H_7N_3O_2S$ (185,21)

| | | | | | |
|---|---|---|---|---|---|
| Ber.: | C 38,91 | H 3,81 | N 22,69 | O 17,28 | S 17,31 |
| Gef.: | C 38,9 | H 3,6 | N 22,8 | O 17,3 | S 17,4 |

**Beispiel 3**

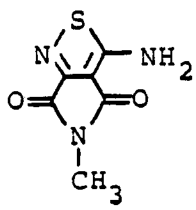

Zu einer Mischung von 62,5 Teilen 3-Amino-2-thiocarbamoyl-N-methylmaleinimid und 300 Teilen Eisessig werden 38,3 Teile einer 30 %-igen wäßrigen Wasserstoffperoxidlösung zugetropft, die Temperatur wird dabei durch Kühlung bei 30 - 40°C gehalten. Anschließend wird die Mischung noch 2 h bei Raumtemperatur nachgerührt, der entstandene Niederschlag abgesaugt, mit Eisessig und dann Wasser gewaschen und getrocknet. Man erhält 54,5 Teile (88 % der Theorie) 5-Aminoisothiazolo-(3,4)-N-methyldicarboximid.

Schmp.: 242°C (Eisessig), IR (KBr): 3420, 3313 ($NH_2$), 1763, 1705, 1611 $cm^{-1}$ (C=O)

$C_6H_5N_3O_2S$ (183,19)

| | | | | | |
|---|---|---|---|---|---|
| Ber.: | C 39,34 | H 2,75 | N 22,94 | O 17,47 | S 17,50 |
| Gef.: | C 39,5 | H 2,9 | N 23,2 | O 17,5 | S 17,4 |

**Beispiel 4**

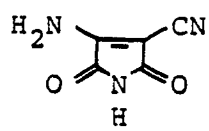

Die Verbindung wird wie in Beispiel 1 beschrieben aus 217 Teilen 2-Cyan-3-methoxy-maleinimid, 1000 Teilen Isopropanol und 26,7 Teilen Ammoniak hergestellt. Ausbeute: 192 Teile (89 % der Theorie) 3-Amino-2-cyan-maleinimid.

Zers.P.: 260°C ($H_2O$), IR (KBr): 3275, 3120 ($NH_2$), 2220 (C≡N), 1782, 1725, 1650 $cm^{-1}$ (C=O)

$C_5H_3N_3O_2$ (137,10)

| | | | | |
|---|---|---|---|---|
| Ber.: | C 43,80 | H 2,21 | N 30,65 | O 23,34 |
| Gef.: | C 43,9 | H 2,3 | N 30,5 | O 23,4 |

**Beispiel 5**

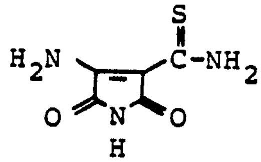

Die Verbindung wird wie in Beispiel 2 beschrieben aus 125 Teilen Phosphorpentasulfid, 600 Teilen n-Butanol und 102,8 Teilen 3-Amino-2-cyan-maleinimid hergestellt. Ausbeute: 61 Teile (48 % der Theorie) 3-Amino-2-thiocarbamoylmaleinimid.

Zers.-P.: 262°C (n.-Butanol), IR (KBr): 3366, 3320, 3191 ($NH_2$), 1762, 1719, 1644, 1610 $cm^{-1}$ (C=O)

$C_5H_5N_3O_2S$ (171,18)

| | | | | | |
|---|---|---|---|---|---|
| Ber.: | C 35,08 | H 2,94 | N 24,55 | O 18,69 | S 18,73 |
| Gef.: | C 35,4 | H 3,1 | N 24,2 | O 18,9 | S 18,7 |

**Beispiel 6**

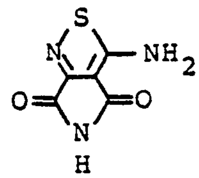

Wie in Beispiel 3 beschrieben erhält man die Verbindung aus 75,2 Teilen 3-Amino-2-thiocarbamoyl-maleinimid, 400 Teilen Essigsäure und 50 Teilen einer 30 %-igen wäßrigen Wasserstoffperoxidlösung. Ausbeute: 68 Teile (91 % der Theorie) 5-Aminoisothiazolo-(3,4)-dicarboximid.
Zers.-P.: 268° C (Methylglykol), IR (KBr): 3419, 3220, 3142 (NH, $NH_2$), 1764, 1714, 1691 $cm^{-1}$ (C=O)

$C_5H_3N_3O_2S$ (169,16)

| | | | | | |
|---|---|---|---|---|---|
| Ber.: | C 35,50 | H 1,79 | N 24,84 | O 16,92 | S 18,95 |
| Gef.: | C 35,4 | H 2,0 | N 24,8 | O 18,5 | S 18,7 |

**Beispiel 7**

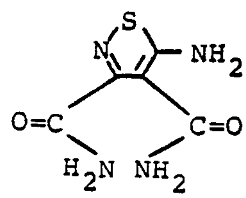

Eine Mischung von 10,1 Teilen 5-Amino-isothiazolo-(3,4)-dicarboximid und 70 Teilen konz. wäßriger Ammoniaklösung wird 2 h bei Raumtemperatur gerührt, der farblose Niederschlag anschließend abgesaugt, mit Wasser gewaschen und getrocknet. Man erhält 9,3 Teile (83 % der Theorie) 5-amino-isothiazol-3,4-dicarboxamid.
Schmp.: 205° C (Wasser), IR (KBr): 3400, 3330, 3275, 3140 ($NH_2$ ), 1680, 1668, 1655 $cm^{-1}$ (C=O)

$C_5B_6N_4O_2S$ (186,19)

| | | | | | |
|---|---|---|---|---|---|
| Ber.: | C 32,25 | H 3,25 | N 30,09 | O 17,19 | S 17,22 |
| Gef.: | C 32,3 | H 3,1 | N 30,4 | O 17,2 | S 17,4 |

**Beispiel 8**

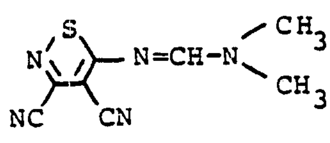

10,1 Teile Phosphoroxytrichlorid werden bei 0°C in 30 Teile Dimethylformamid getropft, anschließend rührt man das Gemisch noch 0,5 h bei dieser Temperatur. Unter Eiskühlung werden dann in diese Lösung 3,7 Teile 5-Amino-isothiazol-3,4-dicarboxamid portionsweise eingetragen. Die Mischung wird noch 2 h bei Raumtemperatur gerührt und anschließend in 100 Teile Eiswasser gegeben. Der Niederschlag wird abgesaugt, mit Wasser gewaschen und getrocknet. Man erhält 3,8 Teile (93 % der Theorie) N,N-Dimethyl-N -(3,4-dicyanisothiazolyl-5)-formamidin.

Schmp.: 173° C (n-Butanol), IR (KBr): 2210 (C≡N), 1625 $cm^{-1}$ (C=N)

$C_8H_7N_5S$ (205,24)

| | | | | |
|---|---|---|---|---|
| Ber.: | C 46,82 | H 3,44 | N 34,12 | S 15,62 |
| Gef.: | C 46,5 | H 3,1 | N 34,1 | S 15,7 |

**Beispiel 9**

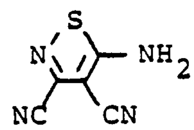

Eine Mischung aus 45,3 Teilen N,N-Dimethyl-N'-(3,4-dicyanisothiazolyl-5)-formamidin, 400 Teilen Ethanol und 60 Teilen konz. Salzsäure wird 2,5 h zum Sieden erhitzt und anschließend in 400 Teile Wasser eingetragen. Der farblose Niederschlag wird abgesaugt, mit Wasser gewaschen und getrocknet. Man erhält 28,6 Teile (87 % der Theorie) 5-Aminoisothiazol-3,4-dicarbonsäurenitril.

Zers.-P.: 240° C (Eisessig) IR (KBr): 3380, 3290, 3190 ($NH_2$), 2210 (C≡N)

$C_5H_2N_4S$ (150,16)

| | | | | |
|---|---|---|---|---|
| Ber.: | C 39,99 | H 1,34 | N 37,31 | S 21,35 |
| Gef.: | C 40,1 | H 1,6 | N 37,3 | S 21,2 |

**Beispiel 10**

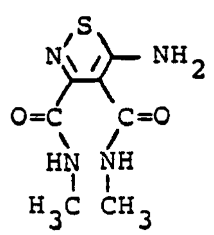

In 50 Teile Methanol wird bei Raumtemperatur Methylamin bis zur Sättigungskonzentration eingeleitet, dann werden 4,6 Teile 5-Amino-isothiazolo-(3,4)-N-methyldicarboximid eingetragen und die Mischung 3 h bei Raumtemperatur gerührt. Die klare Lösung wird dann in 100 Teile Wasser gegeben, der Niederschlag abgesaugt, mit Wasser gewaschen und getrocknet. Man erhält 4,3 Teile (80 % der Theorie) 5-Aminoisothiazol-3,4-di-N-methylcarboxamid.

Schmp.: 222° C (n-Butanol), IR (KBr): 3400, 3350, 3220 ($NH_2$, NH), 1645, 1590 $cm^{-1}$ (C=O)

$C_7H_{10}N_4O_2S$ (214,25)

| | | | | | |
|---|---|---|---|---|---|
| Ber.: | C 39,24 | H 4,70 | N 26,15 | O 14,94 | S 14,97 |
| Gef.: | C 39,5 | H 4,6 | N 26,1 | O 15,2 | S 15,0 |

**Beispiel 11**

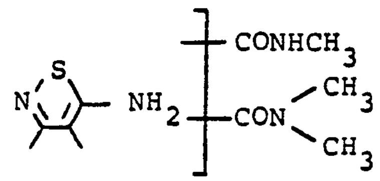

In 50 Teile Methanol wird bei Raumtemperatur Dimethylamin bis zur Sättigungskonzentration eingeleitet, dann werden 10,1 Teile 5-Amino-isothiazolo-(3,4)-N-methyldicarboximid eingetragen und die Mischung 1,5 h bei Raumtemperatur gerührt. Anschließend gibt man 1 Teil Tierkohle zu und filtriert das Gemisch ab. Das Filtrat wird im Vakuum zur Trockne eingeengt und das zurückgebliebene Öl im Vakuum vom restlichen Lösungsmittel befreit. Man erhält 12,1 Teile (97 % der Theorie) eines roten Öls, welches allmählich kristallisiert.

Schmp.: 116° C (Toluol), IR (KBr): 3401, 3255 (NH, $NH_2$), 1636, 1622, 1608, 1595 $cm^{-1}$ (C=O)

$C_8H_{12}N_4O_2S$ (228,27)

| | | | | | |
|---|---|---|---|---|---|
| Ber.: | C 42,09 | H 5,30 | N 24,54 | O 14,02 | S 14,05 |
| Gef.: | C 42,3 | H 5,4 | N 24,2 | O 14,3 | S 13,7 |

**Beispiel 12**

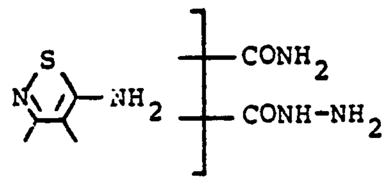

Eine Mischung aus 10,1 Teilen 5-Amino-isothiazolo-(3,4)-dicarboximid, 3,3 Teilen Hydrazinhydrat und 50 Teilen Wasser wird 0,5 h zum Sieden erhitzt. Nach Abkühlen auf Raumtemperatur wird der entstandene Niederschlag abgesaugt, mit Wasser gewaschen und getrocknet. Man erhält 10,5 Teile (87 % der Theorie) einer Verbindung der obigen Formel.

Zers.-P.: 206° C (Wasser), IR (KBr): 3359 , 3301, 3248 (NH, $NH_2$), 1637, 1614 $cm^{-1}$ (C=O)

$C_5H_7N_5O_2S$ (201,21)

| | | | | | |
|---|---|---|---|---|---|
| Ber.: | C 29,85 | H 3,51 | N 34,81 | O 15,90 | S 15,94 |
| Gef.: | C 29,6 | H 3,3 | N 34,5 | O 16,0 | S 16,1 |

**Beispiel 13**

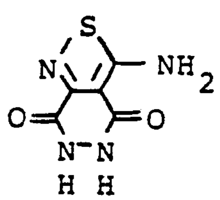

11,5 Teile der Verbindung des Beispiels 12 werden in 200 Teilen Eisessig 10 h zum Sieden erhitzt. Nach dem Abkühlen wird der Niederschlag abgesaugt, mit Eisessig gewaschen und getrocknet. Man erhält 7 Teile (67 % der Theorie) 7-Amino-3,4,5,6-tetrahydro-3,6-dioxo-pyridazino[4,5-c]isothiazol.

Schmp.: > 300° C, IR (KBr): 3417, 3220 (NH, $NH_2$), 2800 sehr breit (NH/OH), 1641 $cm^{-1}$ (C=O)

$C_5H_4N_4O_2S$ (184,18)

| | | | | | |
|---|---|---|---|---|---|
| Ber.: | C 32,61 | H 2,19 | N 30,42 | O 17,37 | S 17,41 |
| Gef.: | C 32,8 | H 2,2 | N 30,0 | O 17,8 | S 17,1 |

## Patentansprüche

1. Verbindungen der Formel

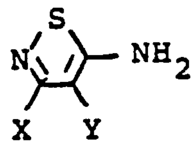

I,

in der

X und Y unabhängig voneinander Carboxyl, $C_1$- bis $C_{12}$-Alkoxycarbonyl, Cyclohexyloxycarbonyl, Allyloxycarbonyl, 2-($C_1$- bis $C_4$-Alkoxy)-ethoxycarbonyl, 3-($C_1$- bis $C_4$-Alkoxyl)-propyloxycarbonyl, 2-Phenoxyethoxycarbonyl, 3-Phenoxypropyloxycarbonyl, Phenylmethoxycarbonyl, 2-Phenylethoxycarbonyl, Phenoxycarbonyl, durch Chlor, Brom, Nitro oder Cyan substituiertes Phenoxycarbonyl, $C_1$- bis $C_4$-Alkylphenoxycarbonyl, Dimethylphenoxycarbonyl, Trimethylphenoxycarbonyl, Carbamoyl, $C_1$- bis $C_{12}$-Alkylcarbamoyl, Cyclohexylcarbamoxyl, Allylcarbamoyl, 2-($C_1$- bis $C_4$-Alkoxy)-ethylcarbamoyl, 3-($C_1$- bis $C_4$-Alkoxy)-propylcarbamoyl, 2-Phenoxyethylcarbamoyl, 3-Phenoxypropylcarbamoyl, Phenylmethylcarbamoyl, 2-Phenylethylcarbamoyl, Phenylcarbamoyl, durch Chlor, Brom, Nitro oder Cyan substituiertes Phenylcarbamoyl, $C_1$- bis $C_4$-Alkylphenylcarbamoyl, Dimethylphenylcarbamoyl, Trimethylphenylcarbamoyl, $C_1$- bis $C_4$-Dialkylcarbamoyl, Methyl-cyclohexylcarbamoyl, Dicyclohexylcarbamoyl,

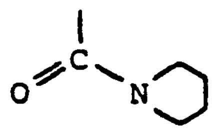 , 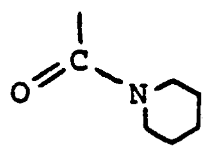 , 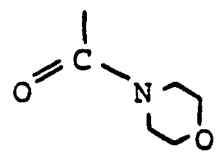 .

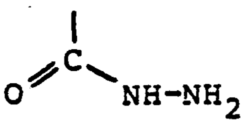 , 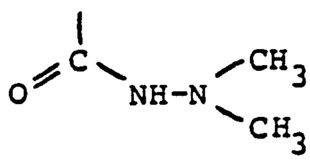 , 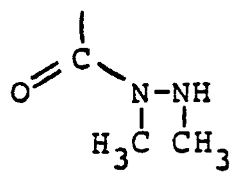 ,

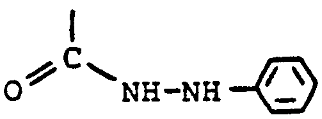 oder 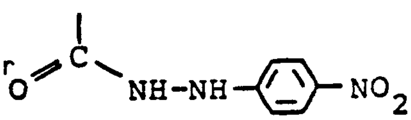 , oder

X und Y zusammen einen Rest der Formel

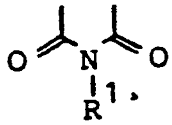 oder 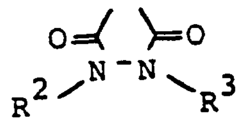

bedeuten, wobei

$R^1$ für Wasserstoff, $C_1$- bis $C_{12}$-Alkyl, Cyclohexyl, Allyl, 2-($C_1$- bis $C_4$-Alkoxy)-ethyl, 3-($C_1$- bis $C_4$-Alkoxy)-propyl, 2-Phenoxyethyl, 3-Phenoxypropyl, Phenylmethyl, Phenylethyl, Phenyl, $C_1$- bis $C_4$-Alkylphenyl, Dimethylphenyl oder Trimethylphenyl oder jeweils durch Chlor, Brom, Cyan oder Nitro substituiertes Phenyl, $C_1$- bis $C_4$-Alkylphenyl, Dimethylphenyl oder Trimethylphenyl, und

$R^2$ und $R_3$ für Wasserstoff oder $C_1$- bis $C_{12}$-Alkyl stehen, wobei einer dieser Reste auch Phenyl oder durch Chlor, Brom, Cyan oder Nitro substituiertes Phenyl bedeuten kann.

2. Verbindungen gemäß Anspruch 1 bei denen

X = Y = CN oder

X und Y = $CONHB^1$ sind,

wobei die Reste $B^1$ gleich oder verschieden sind und

$B^1$ Wasserstoff, $C_1$- bis $C_{12}$-Alkyl oder Phenyl bedeutet.

3. Verbindungen gemäß Anspruch 1, bei denen

X + Y 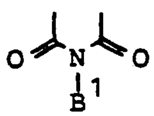 ist und

$B^1$ die in Anspruch 2 angegebene Bedeutung hat.

4. Verbindungen gemäß Anspruch 1, die der Formel

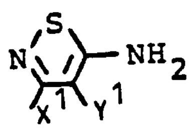

gehorchen, in der einer der Reste $X^1$ und $Y^1$ für den Rest $CONHB^1$ und der andere für Di-($C_1$- bis $C_{12}$-Alkyl)-carbamoyl, Carboxyl, $C_1$- bis $C_{12}$-Alkoxycarbonyl, Phenoxycarbonyl oder einen Rest der Formel

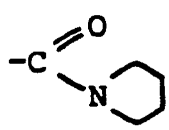 , 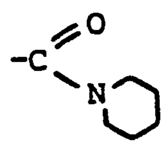 oder 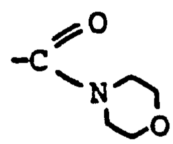

steht und

$B^1$ die angegebene Bedeutung hat.

## Claims

1. A compound of the formula I

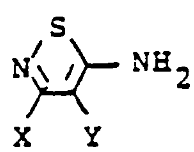 I

where X and Y independently of one another are each carboxyl, $C_1$-$C_{12}$-alkoxycarbonyl, cyclohexyloxycarbonyl, allyloxycarbonyl, 2-($C_1$-$C_4$-alkoxy)-ethoxycarbonyl, 3-($C_1$-$C_4$-alkoxy)-propoxycarbonyl, 2-phenoxyethoxycarbonyl, 3-phenoxypropoxycarbonyl, phenylmethoxycarbonyl, 2-phenylethoxycarbonyl, phenoxycarbonyl which is unsubstituted or substituted by chlorine, bromine, nitro or cyano, $C_1$-$C_4$-alkylphenoxycarbonyl, dimethylphenoxycarbonyl, trimethylphenoxycarbonyl, carbamyl, $C_1$-$C_{12}$-alkylcarbamyl, cyclohexylcarbamyl, allylcarbamyl, 2-($C_1$-$C_4$-alkoxy)-ethylcarbamyl, 3-($C_1$-$C_4$-alkoxy)-propylcarbamyl, 2-phenoxyethylcarbamyl, 3-phenoxypropylcarbamyl, phenylmethylcarbamyl, 2-phenylethylcarbamyl, phenylcarbamyl which is unsubstituted or substituted by chlorine, bromine, nitro or cyano, $C_1$-$C_4$-alkylphenylcarbamyl, dimethylphenylcarbamyl, trimethylphenylcarbamyl, $C_1$-$C_4$-dialkylcarbamyl, methylcyclohexylcarbamyl, dicyclohexylcarbamyl,

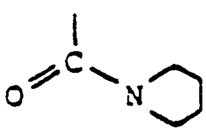 , 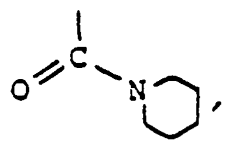 , 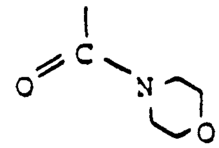

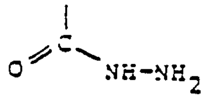 , 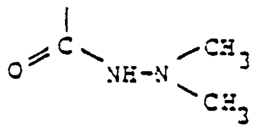 , 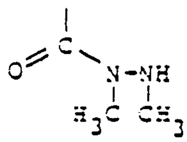 ,

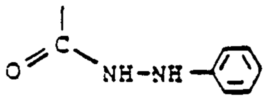 or 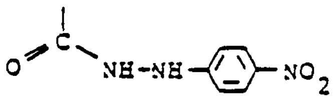 , or

X and Y together form a radical of the formula

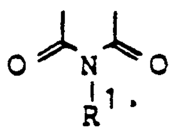 or 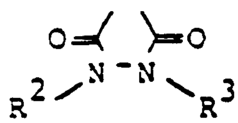

where $R^1$ is hydrogen, $C_1$-$C_{12}$-alkyl, cyclohexyl, allyl, 2-($C_1$-$C_4$-alkoxy)-ethyl, 3-($C_1$-$C_4$-alkoxy)-propyl, 2-phenoxyethyl, 3-phenoxypropyl, phenylmethyl or phenylethyl or is phenyl, $C_1$-$C_4$-alkylphenyl, dimethylphenyl or trimethylphenyl which are each unsubstituted or substituted by chlorine, bromine, cyano or nitro, and $R^2$ and $R^3$ are each hydrogen or $C_1$-$C_{12}$-alkyl, where one of these radicals may furthermore be phenyl which is unsubstituted or substituted by chlorine, bromine, cyano or nitro.

2. A compound as claimed in claim 1, wherein X and Y are each CN or X and Y together form $CONHB^1$, and the radicals $B^1$ are identical or different and are each hydrogen, $C_1$-$C_{12}$-alkyl or phenyl.

3. A compound as claimed in claim 1, wherein X and Y together form

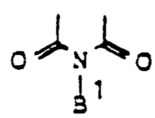

and $B^1$ has the meanings stated in claim 2.

4. A compound as claimed in claim 1, of the formula

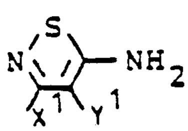

where one of the radicals $X^1$ and $Y^1$ is $CONHB^1$ and the other is di-($C_1$-$C_{12}$-alkyl)-carbamyl, carboxyl, $C_1$-$C_{12}$-alkoxycarbonyl, phenoxycarbonyl or a radical of the formula

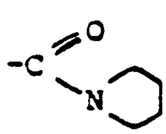 , 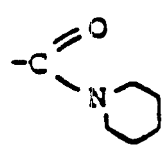 or 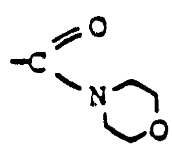

and $B^1$ has the stated meanings.

**Revendications**

1. Composés de formule

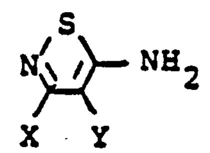 I,

dans laquelle

X et Y représentent indépendamment l'un de l'autre un groupement carboxyle, (alcoxy en $C_1$-$C_{12}$)carbonyle, cyclohexyloxycarbonyle, allyloxycarbonyle, 2-(alcoxy en $C_1$-$C_4$)-éthoxycarbonyle, 3-(alcoxy en $C_1$-$C_4$)-propyloxycarbonyle, 2-phénoxyéthoxycarbonyle, 3-phénoxypropyloxycarbonyle, phénylméthoxycarbonyle, 2-phényléthoxycarbonyle, phénoxycarbonyle, phénoxycarbonyle substitué par des radicaux chloro, bromo, nitro ou cyano, (alkyl en $C_1$- $C_4$)-phénoxycarbonyle, diméthylphénoxycarbonyle, triméthylphénoxycarbonyle, carbamoyle, (alkyl en $C_1$-$C_{12}$)carbamoyle, cyclohexylcarbamoyle, allylcarbamoyle, 2-(alcoxy en $C_1$-$C_4$)éthylcarbamoyle, 3-(alcoxy en $C_1$-$C_4$)propylcarbamoyle, 2-phénoxyéthylcarbamoyle, 3-phénoxypropylcarbamoyle, phénylméthylcarbamoyle, 2-phényléthylcarbamoyle, phénylcarbamoyle, phénylcarbamoyle substitué par des radicaux chloro, bromo, nitro ou cyano, (alkyl en $C_1$-$C_4$)phénylcarbamoyle, diméthylphénylcarbamoyle, triméthylphénylcarbamoyle, di(alkyl en $C_1$-$C_4$)carbamoyle, méthylcyclohexylcarbamoyle, dicyclohexylcarbamoyle,

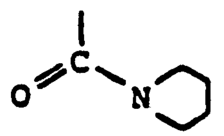 , 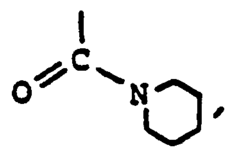 , 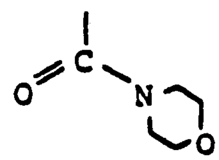 .

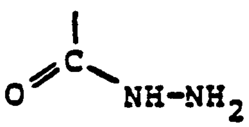 , 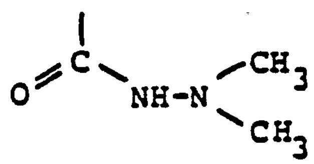 , 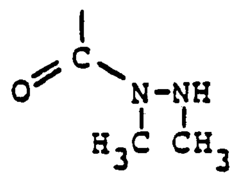 ,

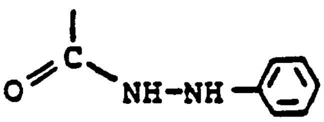 ou 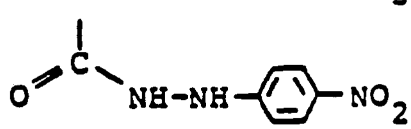

ou bien X et Y représentent ensemble un reste de formule

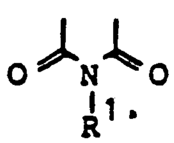 ou 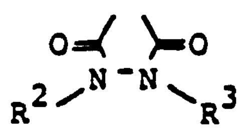

où

$R^1$ est mis pour un atome d'hydrogène ou un radical alkyle en $C_1$-$C_{12}$, cyclohexyle, allyle, 2-(alcoxy en $C_1$-$C_4$)éthyle, 3-(alcoxy en $C_1$-$C_4$)propyle, 2-phénoxyéthyle, 3-phénoxypropyle, phénylméthyle, phényléthyle, phényle, (alkyl en $C_1$-$C_4$)phényle, diméthylphényle ou triméthylphényle, ou phényle, (alkyl en $C_1$-$C_4$)phényle, diméthylphényle ou triméthylphényle substitués par des radicaux chloro, bromo, cyano ou nitro, et

$R^2$ et $R^3$ sont mis pour des atomes d'hydrogène ou des restes alkyle en $C_1$-$C_{12}$, l'un de ces deux restes pouvant aussi être un radical phényle ou phényle substitué par des radicaux chloro, bromo, cyano ou nitro.

2. Composés selon la revendication 1, dans lesquels

X = Y = CN ou

X et Y = $CONHB^1$

où les restes $B^1$ sont identiques ou différents et $B^1$ représente un atome d'hydrogène ou un radical alkyle en $C_1$-$C_{12}$ ou phényle.

3. Composés selon la revendication 1, dans lesquels

X + Y est

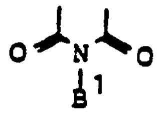

,

et $B^1$ a la signification donnée dans la revendication 2.

4. Composés selon la revendication 1, qui répondent à la formule

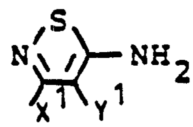

dans laquelle l'un des restes $X^1$ et $Y^1$ est mis pour $CONHB^1$ et l'autre pour un radical di-(alkyl en $C_1$-$C_{12}$)-carbamoyle, carboxy, (alcoxy en $C_1$-$C_{12}$)-carbonyle, phénoxycarbonyle ou de formule

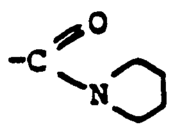

,

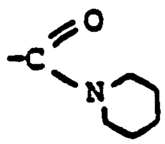

ou

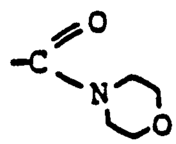

et $B^1$ a la signification donnée ci-dessus.